# FASCICULE DE BREVET EUROPEEN

(11) **EP 2 938 326 B1**
(45) Date de publication et mention de la délivrance du brevet: **20.01.2021**
(21) Numéro de dépôt: 13828846.9
(22) Date de dépôt: 19.12.2013
(51) Int. Cl.: A61K 31/192, A61K 9/00, A61K 9/10, A61K 47/14, A61K 45/06, A61K 31/546, A61P 29/00, A61P 31/00, A61K 47/44

(54) **NOUVELLES COMPOSITIONS PHARMACEUTIQUES VETERINAIRES ET LEUR PROCEDE DE PREPARATION**
NEUARTIGE VETERINÄRMEDIZINISCHE PHARMAZEUTISCHE ZUSAMMENSETZUNGEN UND VERFAHREN ZUR HERSTELLUNG DAVON
NOVEL VETERINARY PHARMACEUTICAL COMPOSITIONS AND METHOD FOR THE PRODUCTION THEREOF

(30) Priorité: 27.12.2012 FR 1262821
(43) Date de publication de la demande: 04.11.2015
(73) Titulaire: VIRBAC, 06516 Carros (FR)
(72) Inventeur: DELHOM, Nathalie, F-06140 Vence (FR)
(74) Mandataire: Gevers & Orès
(86) Numéro de dépôt international: PCT/IB2013/061141
(87) Numéro de publication internationale: WO 2014/102679

(56) Documents cités:
- WO-A1-98/41207
- WO-A2-2010/059717
- US-A- 4 614 741
- US-A1- 2004 146 537
- INGLE D J ET AL: "The effect of aspirin upon the glycosuria of partially depancreatized rats in the presence and absenre of the adrenal glands", ENDOCRINOLOGY 1953, vol. 52, no. 4, 1953, pages 403-406, XP002712361, ISSN: 0013-7227

## Description

La présente invention concerne le domaine de la chimie thérapeutique vétérinaire et plus particulièrement celui des médicaments.

Elle a pour objet de nouvelles compositions pharmaceutiques vétérinaires à action anti-inflammatoire.

Elle a spécifiquement pour objet des compositions pharmaceutiques vétérinaires à administration parentérale renfermant à titre de principe actif un agent anti-inflammatoire de type non stéroïdien (AINS) en association ou en mélange avec un excipient ou un véhicule approprié pour l'usage vétérinaire.

Elle concerne une suspension huileuse à administration parentérale contenant un anti-inflammatoire non stéroïdiens (AINS) portant une fonction acide, à savoir le kétoprofène. L'objet de l'invention est tel que défini dans les revendications.

Le kétoprofène, ou acide 2-(3-benzoylphényl) propionique, est connu depuis 1972 comme médicament anti-inflammatoire non-stéroïdien. Il est commercialisé depuis longue date sous forme de comprimés à action immédiate ou à action prolongée et sous forme injectable prête à l'emploi.

Il est couramment utilisé pour ses propriétés analgésiques, antipyriques et anti-inflammatoires. Il agit en inhibant la synthèse des prostaglandines et des leucotriènes.

Les formulations injectables actuellement commercialisées utilisent le kétoprofène sous forme soluble, c'est le cas des produits commerciaux Ketofen® et Comforion® dans lesquels un sel de kétoprofène est formulé dans une solution aqueuse injectable. Le WO 99/27906 décrit également des solutions injectables à libération prolongée à base d'huile de ricin hydrogénée dans lesquelles le kétoprofène est solubilisé.

D'autres documents, la Demande US 2004/0146537 et le Brevet US 5,665,384, décrivent des formulations de kétoprofène dans de l'huile notamment sous forme de sel, soit son sel de sodium, soit un sel azoté tel que le sel d'arginine, de lysine ou de N-méthylglucamine, mais il s'agit là de compositions destinées à une administration orale Le document US4,614,741 décrit une suspension huileuse de kétoprofène mais ce document ne donne aucune information sur la taille des particules de principe actif.

Les soins prodigués aux animaux de rente, bovins, caprins, ovins et porcins, le sont principalement par les voies d'administration parentérale (incluant l'injection intramusculaire, intradermique, intralésionnelle, intraarticulaire ou sous-cutanée). Afin d'éviter de pratiquer plusieurs injections consécutivement, il est préférable de proposer au praticien une formulation de kétoprofène compatible avec des formulations vétérinaires injectables telles que des suspensions huileuses injectables d'antibiotique. Par compatible, on entend que la formulation de kétoprofène puisse être mélangée par le praticien avec d'autres compositions vétérinaires injectables dans une même seringue avant de pratiquer l'injection à l'animal ou bien que la formulation de kétoprofène comprenne également au moins un autre principe actif tel qu'un antibiotique.

La Demande Internationale WO 2010/059717 décrit une suspension huileuse injectable de ceftiofur, en particulier, de chlorhydrate de ceftiofur, dans une huile biocompatible comprenant en outre du kétoprofène et au moins un conservateur, un agent dispersant et un agent de floculation. Une telle formulation présente toutefois un intérêt limité en ce qu'en raison du nombre important d'ingrédients qu'elle comprend, il sera difficile d'y formuler des concentrations importantes d'actifs sans augmenter le volume de l'huile biocompatible, et donc le volume total de la composition à injecter, ce qui peut rendre son administration difficile. Par ailleurs, la présence de nombreux excipients augmente le risque d'incompatibilité entre les constituants et de toxicité chez l'animal de destination.

De plus, pour éviter des interactions entre excipients dans une formulation, il est préférable de limiter leur quantité et leur diversité. En effet, la combinaison d'excipients peut conduire à des comportements aléatoires de la suspension comme des solubilisations partielles du principe actif non contrôlées conduisant à des actions thérapeutiques différentes ou à des effets de gradient dans la suspension ayant les mêmes conséquences. De plus, moins il y a de composants comme les excipients, moins il y a de risque d'incompatibilité de la suspension lors d'une association avec d'autres formulations injectables.

La Demanderesse s'est donnée pour but la mise au point d'une formulation injectable d'AINS qui résout les problèmes suivants :
- cette formulation doit être telle qu'elle ne conduit pas à une libération retardée ou prolongée de l'AINS ; et
- elle doit pouvoir être associée par un praticien à d'autres formulations vétérinaires huileuses injectables sans risque.

Ainsi, dans le cadre de ses travaux visant à améliorer la formulation du kétoprofène pour une administration parentérale, la Demanderesse a constaté que l'utilisation des formes salifiées des AINS portant une fonction acide telles que leur sel sodique ou leur sel de lysine conduit à une instabilité chimique de l'AINS dans la suspension. Au contraire, les AINS portant une fonction acide libre peuvent être avantageusement formulés en suspension dans une huile.

Elle a également observé que l'utilisation d'un AINS sous forme micronisée telle que la répartition granulométrique est contrôlée et la taille des particules est réduite, améliore de façon inattendue la stabilité physique et chimique de la suspension huileuse et de l'actif.

Il est connu que la vitesse de sédimentation de particules en suspension augmente avec la taille desdites particules et que la remise en suspension des particules sédimentées est d'autant plus facile que la taille de ces particules est grosse. Il est également connu que, si les particules de petites tailles ont tendance à sédimenter plus lentement, le sédiment formé avec des petites particules comprend des interactions particulaires plus fortes et conduit à un phénomène appelé "caking" ; dans ce cas, la remise en suspension est très difficile car il faut rompre les interactions entre les particules pour obtenir une suspension homogène.

Ainsi, dans le cas présent, la stabilité de la suspension huileuse et le fait que les particules micronisées d'AINS ne forment pas un sédiment dense et difficile à remettre en suspension apparaissent comme remarquables.

Il ressort de ces travaux que la Demanderesse a déterminé la répartition granulométrique des particules micronisées la plus adaptée pour mettre en suspension dans un véhicule huileux des particules de kétoprofène ; La taille des particules micronisées permet de façon inattendue d'avoir à la fois une grande stabilité physique de la suspension et une bonne remise en suspension et cela sans préjudice d'une bonne seringabilité et de la stabilité microbiologique de la suspension huileuse.

La Demanderesse a ainsi mis en évidence qu'une telle suspension huileuse d'AINS micronisé, en l'absence de tout autre excipient que le véhicule huileux :
- permet une libération plasmatique de l'AINS rapide, similaire à celle obtenue après injection d'une solution aqueuse de sel de kétoprofène comme le Ketofen® ou le Comforion®;
- est stable chimiquement ;
- est stable d'un point de vue microbiologique ; c'est-à-dire qu'une suspension huileuse de kétoprofène stérile reste stérile même lors de plusieurs réemplois après une première ouverture (voir l'exemple 3b) ;
- présente une très bonne stabilité physique permettant d'excellentes remises en suspension et seringabilité (voir exemple 3c).

Ainsi la présente invention se rapporte à une composition huileuse d'un anti-inflammatoire non stéroïdien (AINS) pour une administration parentérale comprenant :
- au moins un AINS portant une fonction acide ; et
- un véhicule huileux biocompatible ; également désigné par huile dans ce qui suit ;
ladite composition étant caractérisée en ce que :
- ledit AINS est sous forme d'acide libre ;
- de préférence, ledit AINS est en suspension dans ledit véhicule huileux avec des particules ayant une taille répondant aux spécifications suivantes : un diamètre médian D (v,0.5), valeur pour laquelle 50% de la distribution est inférieure ou égale, de 25 µm et une valeur D (v,0.9), pour laquelle 90% de la distribution est inférieure ou égale, de 80 µm ; et
- ladite composition ne contient aucun agent capable de solubiliser ledit AINS.

Dans les travaux qui ont conduit à la mise au point de la composition huileuse d'AINS selon l'invention, la Demanderesse a constaté que la formulation de l'AINS en suspension dans l'huile (ou véhicule huileux) était essentielle pour l'obtention des propriétés avantageuses de ladite composition, en particulier, pour sa stabilité mais aussi pour l'observation du profil plasmatique recherché (à action immédiate).

En effet, l'emploi de composés capables de solubiliser l'AINS peut conduire à des formulations qui présentent une activité thérapeutique aléatoire car, lors de l'injection, le principe actif repassera potentiellement, au moins en partie, sous forme solide ce qui modifiera sa mise à disposition dans l'organisme.

Il convient toutefois de souligner que la formulation d'une suspension pose des difficultés et qu'un objectif de la présente invention est de mettre au point une suspension ne prenant pas en masse et se remettant facilement en suspension ; plus particulièrement, cette suspension doit être homogène et ne doit pas coaguler, ne doit pas conduire à la formation de masses gélatineuses, ni conduire à la formation d'agglomérats, masses compactes difficilement divisibles. Enfin, la suspension doit être chimiquement stable vis-à-vis d'elle-même mais aussi vis-à-vis des autres formulations qui peuvent lui être associées.

Les suspensions sont des systèmes dispersés dans lesquels une phase interne, qui est un solide très finement divisé (également appelées par la suite particules), cette phase est aussi désignée phase à suspendre, est distribuée de manière uniforme après une agitation mécanique dans une phase externe appelée milieu suspenseur ou véhicule. La phase interne est une distribution homogène de particules appartenant à un domaine spécifique de taille.

Les véhicules pharmaceutiquement acceptables les plus couramment utilisés pour les suspensions parentérales sont des huiles d'origine naturelle ou synthétique.

Quand cela s'avère nécessaire, le maintien en suspension des particules solides dans le véhicule suspenseur est amélioré par l'ajout d'un ou plusieurs agents suspenseurs.

La stabilité d'une suspension fait intervenir différents facteurs ; le maintien de cette stabilité est souvent compliqué par le fait que les facteurs qui affectent la stabilité physique peuvent aussi affecter la stabilité chimique des composants de ladite suspension. Ceci peut être dû au fait qu'une suspension comprend des matières dans plusieurs états physiques, solide et liquide. Au cours de la vie de la suspension, la dispersion des matières solides dans le milieu liquide va évoluer. Cette évolution conduit le milieu hétérogène à être soumis à différents mécanismes qui vont modifier aussi bien physiquement que chimiquement la suspension.

Un moyen de préparer une suspension huileuse satisfaisante est l'utilisation de suspension pharmaceutique floculée ; il s'agit d'une suspension dans laquelle les particules solides sont susceptibles de former des agrégats lorsque la suspension est au repos mais qui sont facilement remises en suspension par une agitation mécanique.

Les définitions suivantes seront utiles pour différencier trois termes étroitement liés : floculation, agglomération et coagulation.

La floculation se réfère à la formation d'agrégats, tels des flocons, réversibles, constitués de particules discrètes maintenues entre elles par une structure en réseau soit par adsorption entre les particules, soit par le jeu des forces attractives de Van der Waals au détriment des forces répulsives.

Par agglomération, il faut comprendre qu'un grand nombre de particules sont regroupées étroitement en masse compacte très difficilement dissociables.

La coagulation se réfère à une prise en masse solide ou semi-solide de particules dans un état liquide, quelquefois sous la forme d'un gel fluide.

Les principaux avantages d'une suspension floculée sont les suivants :
1. les agrégats ont tendance à se diviser très aisément par simple agitation ou léger cisaillement du récipient contenant la suspension, voire même par simple passage au travers d'un orifice comme une aiguille pour injection ;
2. par opposition aux systèmes défloculés, les suspensions floculées pourront sédimenter rapidement et être remises sous forme de suspension homogène très facilement même après une longue période de conservation au repos ;
3. une suspension floculée peut être produite selon n'importe quel procédé même si ce procédé requiert la mise en œuvre de conditions aseptiques de formulation des constituants du véhicule pharmaceutiquement acceptable.

Plusieurs procédés permettent d'obtenir des suspensions pharmaceutiques floculées. Le choix dépendra des propriétés du principe actif envisagé mais aussi de la qualité de suspension désirée.

Des auteurs ont décrit des procédés de préparation de telles suspensions huileuses floculées, par exemple par ajout d'eau (US 5,736,151) ou d'autres solvants organiques comme le glycérol, le propylène glycol, polyéthylène glycol, des alcools comme l'éthanol (US 4,902,683 et WO 2004/014390) ; cependant, ces composés étant capables de solubiliser les formes acides libres des AINS portant une fonction acide, ils sont préférentiellement à éviter dans le cadre de la présente invention.

La Demanderesse est parvenue à préparer une suspension huileuse d'AINS stable avec une répartition homogène des particules grâce au choix d'une taille des particules d'AINS particulière et grâce à l'homogénéité de la taille de ces particules. La taille des particules d'AINS répond aux spécifications suivantes : une valeur D (v,0.5) inférieure ou égale à 25 µm et une valeur D (v,0.9) inférieure ou égale à 80 µm. La taille des particules d'AINS peut également être exprimée comme suit : elle est comprise entre 1 et 100 µm, et plus préférentiellement centrée autour de 10 µm, de 15 µm, de 20 µm, ou de 25 µm.

Les valeurs suivantes pour la taille des particules d'AINS en suspension dans l'huile seront préférées :

| D (v,0.5) / D (v,0.9) |
|---|
| 15 µm / 80 µm |
| 20 µm / 80 µm |
| 25 µm / 80 µm |
| |

La composition selon l'invention comprend entre 1 et 30 % en poids/volume (en poids par rapport au volume de la composition finale) d'au moins un anti-inflammatoire non stéroïdien (AINS) portant une fonction acide ; avantageusement entre 10 et 20 % en poids/volume et, plus avantageusement encore, 15 % en poids/volume.

Sauf indication contraire, les concentrations mentionnées dans ce qui suit sont exprimées en pourcentage du poids par rapport au volume total de la composition (% en poids/volume).

Cette teneur en kétoprofène, permet d'obtenir le meilleur compromis entre l'efficacité thérapeutique de la composition selon l'invention et du volume à injecter respectant ainsi, autant que possible, le bien être de l'animal à traiter.

Le véhicule huileux biocompatible est choisi préférentiellement parmi les huiles végétales comme par exemple l'huile de palme, l'huile de maïs, l'huile de coton, l'huile de tournesol, l'huile d'arachide, l'huile d'olive, l'huile de soja, l'huile de carthame, l'huile de coprah, l'huile de sésame, ou parmi les huiles végétales semi-synthétiques obtenues par fractionnement et/ou hydrolyse et/ou estérification totale des huiles végétales naturelles comme par exemple les triglycérides d'acide gras issus d'huiles végétales, comme les triglycérides des acides caprylique, caprique, linoléïque, succinique (vendues sous les dénominations commerciales Miglyol® 810, 812, 818, 820, 829), les esters du propylène glycol et d'acide gras issus d'huile végétale comme les esters du propylène glycol et des acides caprylique et caprique (vendues sous les dénominations commerciales Miglyol® 840), ainsi que leur mélange. Préférentiellement, le véhicule huileux est de l'huile de coton.

Selon un mode de réalisation particulier de l'invention, la composition huileuse d'AINS selon l'invention ne contient aucun agent liquide ajouté capable de solubiliser dans l'huile ledit composé anti-inflammatoire non stéroïdien ; en particulier, ces agents exclus de la composition huileuse d'AINS selon l'invention sont : l'eau, le propylène glycol, le glycérol, le polyéthylène glycol, des alcools en C₁ à C₆ comme l'éthanol, ainsi que les alcools aromatiques, c'est-à-dire des composés comprenant une fonction hydroxyle liée directement ou via une liaison alkyle à un noyau aromatique tel qu'un noyau phényle éventuellement substitué, tels que l'alcool benzylique ou l'alcool phényléthylique, ou un noyau naphtyle éventuellement substitué.

Selon une variante de ce mode de réalisation de la présente invention, la composition huileuse d'AINS selon l'invention ne contient aucun autre composé que l'AINS portant une fonction acide et le véhicule huileux biocompatible.

Selon un autre mode de réalisation particulier de l'invention, il est possible d'ajouter à la composition huileuse d'AINS au moins un agent mouillant.

Les agents mouillants utilisables selon l'invention sont choisis parmi : les huiles de ricin hydrogénées polyoxyéthylénées, les huiles de ricin polyoxyéthylénées, les huiles végétales hydrogénées polyoxyéthylénées, les huiles végétales hydrogénées polyoxyéthylénées, le monostéarate de glycérol, les huiles de ricin polyoxyéthylénées, les esters d'acides gras polyoxyéthylénés et de sorbitan (vendus sous les dénominations commerciales Montanox 20DF, 40DF, 60DF, 80 VG DF, 81, 85 VG DF), les esters d'acides gras et de sorbitan (vendus sous les dénominations commerciales Montane® 20, 40, 60, 65, 70, 80 VG PHA, 85 VG), la lécithine et ses dérivés de soja ou d'oeuf comme par exemple la phosphatidylcholine (vendue sous la dénomination commerciale Phospholipon® 90G), la phosphatidylcholine hydrogénée (vendue sous la dénomination commerciale Phopholipon® 80H, Phospholipon® 90H), la lysophosphatidylcholine, la lysophosphatidylcholine hydrogénée, ainsi que leur combinaison.

Les agents mouillants sont utilisés à des concentrations comprises entre 0,01 et 1% en poids/volume ; avantageusement entre 0,05 et 0,25 % en poids/volume, et plus avantageusement encore, à 0,20 % en poids/volume. L'agent mouillant est préférentiellement le monooleate de sorbitan (Montane 80 VG PHA) et/ou la phosphatidylcholine hydrogénée de soja (Phospholipon® 90H).

Selon un autre mode de réalisation de la présente invention, la composition huileuse d'AINS selon l'invention ne contient aucun autre composé que l'AINS portant une fonction acide, le véhicule huileux biocompatible et un ou plusieurs agents mouillants.

Avantageusement, les compositions selon l'invention peuvent aussi comprendre des composés supplémentaires solides tels que des agents antimicrobiens, des agents antioxydants, des excipients et/ou ou adjuvants physiologiquement acceptables. A titre d'exemples d'antioxydants, on peut citer sans limitation le butylhydroxyanisol (BHA), butylhydroxytoluène (BHT) et les mélanges de ceux-ci.

Selon encore une autre disposition préférée de l'invention, la composition huileuse à administration parentérale selon l'invention est utilisée comme médicament pour un traitement de l'inflammation (action anti-inflammatoire), de la douleur (action antalgique) et/ou de la fièvre (action antipyrique) des animaux d'élevage tels que les bovins ou les porcins ; notamment, en complément d'une antibiothérapie spécifique. Elle peut aussi avantageusement être utilisée comme médicament pour des animaux de compagnies, tels que les chiens et les chats.

La composition huileuse d'AINS à administration parentérale selon l'invention pourra être administrée en tant que seul traitement directement à l'animal ou associée extemporanément avec une composition huileuse d'antibiotique, soit par mélange des compositions avant administration à l'animal, soit par administration séquentielle de chaque composition à l'animal.

La composition huileuse d'AINS selon l'invention s'avère particulièrement adaptée au mélange et à l'administration avec une suspension huileuse d'antibiotique telle qu'une suspension huileuse de chlorhydrate de ceftiofur comme le produit commercial EXCENEL® RTU ou une suspension huileuse de ceftiofur cristallisé acide libre comme le produit commercial EXCEDE® ou une suspension huileuse d'amoxicilline comme les produits commerciaux VETRIMOXIN®, SURAMOX® ou POTENCIL® (suspension injectable contenant aussi de la Colistine sous forme de sulfate). Selon un autre de ses objets, l'invention se rapporte à une composition huileuse d'AINS à administration parentérale qui comprend d'autres principes actifs médicamenteux en vue d'élargir le spectre d'activité de la composition, d'améliorer ainsi l'activité thérapeutique en traitant simultanément et en une seule injection plusieurs pathologies.

Ainsi, selon encore une autre disposition, la présente invention se rapporte à une composition huileuse d'un anti-inflammatoire non stéroïdien (AINS) et d'agent anti-infectieux pour une administration parentérale comprenant :
- au moins un AINS portant une fonction acide ;
- au moins un agent anti-infectieux tels que les antibiotiques bêta-lactamines comme par exemple les benzylpénicillines ou les aminopénicillines ou les céphalosporines ; et
- un véhicule huileux biocompatible ;
ladite composition étant caractérisée en ce que :
- ledit AINS est sous forme d'acide libre ;
- ledit AINS et ledit agent anti-infectieux sont en suspension dans ledit véhicule huileux avec des particules ayant une taille répondant aux spécifications suivantes : un diamètre médian D (v,0.5), valeur pour laquelle 50% de la distribution est inférieure ou égale, de 25 µm, préférentiellement de 20 µm, préférentiellement de 15 µm et encore plus préférentiellement de 10 µm et une valeur D (v,0.9), pour laquelle 90% de la distribution est inférieure ou égale, de 80 µm, préférentiellement de 75 µm, préférentiellement de 70 µm et encore plus préférentiellement de 65 µm ; et
- ladite composition ne contient aucun agent capable de solubiliser ledit composé anti-inflammatoire non stéroïdien.

Selon encore une autre disposition préférée de l'invention, la composition huileuse à administration parentérale selon l'invention comprenant au moins un AINS portant une fonction acide et au moins un agent anti-infectieux est utilisée comme médicament pour un traitement de l'infection et de l'inflammation (action anti-inflammatoire), de la douleur (action antalgique) et/ou de la fièvre (action antipyrique) des animaux d'élevage tels que les bovins ou les porcins. Elle peut aussi avantageusement être utilisée comme médicament pour des animaux de compagnies, tels que les chiens et les chats.

L'invention a aussi pour objet la composition huileuse à administration parentérale selon l'invention comprenant au moins un AINS portant une fonction acide et au moins un agent anti-infectieux tels que les antibiotiques bêta-lactamines comme par exemple les benzylpénicillines ou les aminopénicillines ou les céphalosporines dans un véhicule huileux biocompatible ;
ladite composition étant caractérisée en ce que :
- ledit AINS est sous forme d'acide libre ;
- ledit AINS et ledit agent anti-infectieux sont en suspension dans ledit véhicule huileux avec des particules ayant une taille répondant aux spécifications suivantes : un diamètre médian D (v,0.5), valeur pour laquelle 50% de la distribution est inférieure ou égale, de 25 µm, préférentiellement de 20 µm, préférentiellement de 15 µm et encore plus préférentiellement de 10 µm et une valeur D (v,0.9), pour laquelle 90% de la distribution est inférieure ou égale, de 80 µm, préférentiellement de 75 µm, préférentiellement de 70 µm et encore plus préférentiellement de 65 µm ; et
- ladite composition ne contient aucun agent capable de solubiliser ledit composé anti-inflammatoire non stéroïdien ;
pour utilisation dans le traitement des maladies infectieuses des animaux d'élevage tels que les bovins, ovin et caprins ou des animaux de compagnies tels que les chiens et chats.

L'invention a aussi pour objet l'utilisation à des fins antipyrique et antiinflammatoire de la composition huileuse à administration parentérale selon l'invention comprenant au moins un AINS portant une fonction acide et au moins un agent anti-infectieux tels que les antibiotiques bêta-lactamines comme par exemple les benzylpénicillines ou les aminopénicillines ou les céphalosporines dans un véhicule huileux biocompatible ;
ladite composition étant caractérisée en ce que :
- ledit AINS est sous forme d'acide libre ;
- ledit AINS et ledit agent anti-infectieux sont en suspension dans ledit véhicule huileux avec des particules ayant une taille répondant aux spécifications suivantes : un diamètre médian D (v,0.5), valeur pour laquelle 50% de la distribution est inférieure ou égale, de 25 µm, préférentiellement de 20 µm, préférentiellement de 15 µm et encore plus préférentiellement de 10 µm et une valeur D (v,0.9), pour laquelle 90% de la distribution est inférieure ou égale, de 80 µm, préférentiellement de 75 µm, préférentiellement de 70 µm et encore plus préférentiellement de 65 µm ; et
- ladite composition ne contient aucun agent capable de solubiliser ledit composé anti-inflammatoire non stéroïdien.

De la même façon que pour les compositions huileuses d'AINS et d'agent anti-infectieux selon l'invention, le véhicule huileux biocompatible est choisi parmi les huiles végétales comme par exemple l'huile de palme, l'huile de maïs, l'huile de coton, l'huile de tournesol, l'huile d'arachide, l'huile d'olive, l'huile de soja, l'huile de carthame, l'huile de coprah, l'huile de sésame, ou parmi les huiles végétales semi-synthétiques obtenues par fractionnement et/ou hydrolyse et/ou estérification totale des huiles végétales naturelles comme par exemple les triglycérides d'acide gras issus d'huiles végétales, comme les triglycérides des acides caprylique, caprique, linoléïque, succinique (vendues sous les dénominations commerciales Miglyol® 810, 812, 818, 820, 829), les esters du propylène glycol et d'acide gras issus d'huiles végétales comme les esters du propylène glycol et des acides caprylique et caprique (vendues sous les dénominations commerciales Miglyol® 840), ainsi que leur mélange. Préférentiellement, le véhicule huileux est de l'huile de coton.

L'anti-inflammatoire non stéroïdien (AINS) est présent à une concentration comprise entre 1 et 30 % en poids/volume (en poids par rapport au volume de la composition finale) portant une fonction acide ; avantageusement entre 10 et 20 % en poids/volume et, plus avantageusement encore, 15 % en poids/volume.

L'AINS utilisé dans l'invention est le kétoprofène.

Comme expliqué précédemment, il est avantageux d'utiliser des particules d'AINS en suspension ayant une taille répondant aux spécifications suivantes : un diamètre médian D (v,0.5), valeur pour laquelle 50% de la distribution est inférieure ou égale, de 25 µm et une valeur D (v,0.9), pour laquelle 90% de la distribution est inférieure ou égale, de 80 µm. Autrement exprimé, ledit AINS est en suspension dans l'huile avec des particules ayant une taille comprise entre 1 et 100 µm, et plus préférentiellement centrée autour de 10 µm, de 15 µm, de 20 µm, ou de 25 µm.

Les valeurs suivantes pour la taille des particules d'AINS en suspension dans l'huile seront préférées :

| D (v,0.5) / D (v,0.9) |
|---|
| 15 µm / 80 µm |
| 20 µm / 80 µm |
| 25 µm / 80 µm |

Selon un mode de réalisation avantageux desdites compositions huileuses d'AINS et d'agent anti-infectieux à administration parentérale de l'invention, l'agent anti-infectieux est un antibiotique de la famille des bêta-lactamines ; de préférence, une céphalosporine et plus particulièrement, le chlorhydrate de ceftiofur.

Les compositions selon l'invention comprennent entre 1 et 20 % en poids/volume d'agent anti-infectieux, avantageusement entre 1 et 10 % en poids/volume, et plus particulièrement 5 % en poids/volume.

Pour assurer la stabilité de la suspension, il est important que ledit agent anti-infectieux qui est également en suspension dans le véhicule huileux biocompatible se présente sous forme de particule ayant une taille proche celle des particules d'AINS, c'est-à-dire une taille répondant aux spécifications suivantes : un diamètre médian D (v,0.5), valeur pour laquelle 50% de la distribution est inférieure ou égale, de 15 µm, préférentiellement de 10 µm et encore plus préférentiellement de 5 µm et une valeur D (v,0.9) pour laquelle 90% de la distribution est inférieure ou égale, de 65 µm, préférentiellement de 50 µm et encore plus préférentiellement de 35 µm. Autrement exprimé, ledit agent anti-infectieux est en suspension dans l'huile avec des particules ayant une taille moyenne comprise entre 1 et 100 µm, préférentiellement centrée entre 2 et 20 µm et plus particulièrement centrée autour de 1 µm, de 2 µm, de 3 µm, de 4 µm, de 5 µm, de 6 µm, de 7 µm ou de 8 µm.

Plus particulièrement, les compositions selon l'invention sont telles que le ratio de la taille moyenne des particules d'agent anti-infectieux par rapport à la taille moyenne des particules d'AINS est compris entre 1:3 et 1:30, préférentiellement 1:5 et 1:15 et encore préférentiellement entre 1:5 et 1:8.

Selon un mode de réalisation particulier de l'invention, la composition huileuse d'AINS et d'agent anti-infectieux selon l'invention ne contient aucun agent capable de solubiliser dans l'huile les deux principes actifs ; en particulier, ces agents exclus de la composition huileuse d'AINS selon l'invention sont: l'eau, le propylène glycol, le glycérol, le polyéthylène glycol, des alcools en C₁ à C₆ comme l'éthanol, ainsi que les alcools aromatiques, c'est-à-dire des composés comprenant une fonction hydroxyle liée directement ou via une liaison alkyle à un noyau aromatique tel qu'un noyau phényle comme l'alcool benzylique ou l'alcool phényléthylique, ou un noyau naphtyle éventuellement substitué.

Selon un mode de réalisation particulièrement préféré, la composition huileuse d'AINS et d'agent anti-infectieux selon l'invention ne contient pas d'alcool benzylique.

Selon un autre mode de réalisation particulier de l'invention, il est possible d'ajouter à la composition huileuse d'AINS et d'agent anti-infectieux au moins un agent mouillant.

Les agents mouillants utilisables selon l'invention sont choisis parmi : les huiles de ricin hydrogénées polyoxyethylénées, les huiles de ricin polyoxyethylénées, les huiles végétales hydrogénées polyoxyethylénées, les huiles végétales hydrogénées polyoxyethylénées, le monostéarate de glycérol, les esters d'acides gras polyoxyéthylénés et de sorbitan (vendus sous les dénominations commerciales Montanox 20DF, 40DF, 60DF, 80 VG DF, 81, 85 VG DF), les esters d'acides gras et de sorbitan (vendus sous les dénominations commerciales Montane® 20, 40, 60, 65, 70, 80 VG PHA, 85 VG), la lécithine et ses dérivés de soja ou d'œuf comme par exemple la phosphatidylcholine (vendue sous la dénomination commerciale Phospholipon® 90G), la phosphatidylcholine hydrogénée (vendue sous la dénomination commerciale Phopholipon® 80H, Phospholipon® 90H), la lysophosphatidylcholine, la lysophosphatidylcholine hydrogénée, ainsi que leur combinaison.

Les agents mouillants sont utilisés à des concentrations comprises entre 0,01 et 1% en poids/volume ; avantageusement entre 0,05 et 0,25% en poids/volume, et plus avantageusement encore, à 0,2% en poids/volume. L'agent mouillant est préférentiellement le monooleate de sorbitan (Montane 80 VG PHA) et/ou la phosphatidylcholine hydrogénée de soja (Phospholipon® 90H).

L'impact du chlorhydrate de ceftiofur dans la formulation selon l'invention sur les concentrations plasmatiques en kétoprofène a été évalué en comparant la cinétique du kétoprofène après l'administration de la composition huileuse selon l'invention contenant du chlorhydrate de ceftiofur et du kétoprofène et une composition de chlorhydrate de ceftiofur seul. Il a été observé de manière inattendue que le chlorhydrate de ceftiofur a augmenté la biodisponibilité du kétoprofene. Du point de vue clinique, les activités anti-inflammatoires et antipyrétique de la combinaison ont été montrées comme étant comparables à celles du produit de référence (Ketofen ®, Mérial), sans aucun problème de sécurité supplémentaire.

Selon un mode de réalisation particulier, la présente invention se rapporte à l'une des compositions suivantes :

| **Nom** | **% p/V** | **quantité pour 1 ml** |
|---|---|---|
| **Agent actif** | | |
| Kétoprofène | 15 % | 150 mg |

| **Excipients** | | |
|---|---|---|
| Oléate de sorbitan | 0.15 % | 1.5 mg |
| Phosphatidylcholine hydrogénée, grade 90 | 0.05 % | 0.5 mg |
| huile de coton | jusqu'à 100 % | jusqu'à 1 ml |

| **Agents actifs** | | |
|---|---|---|
| Ceftiofur (sous forme de Chlorhydrate) | 5 % | 50 mg |
| Kétoprofène | 15 % | 150 mg |

| **Excipients** | | |
|---|---|---|
| Oléate de sorbitan | 0.15 % | 1.5 mg |
| Phosphatidylcholine hydrogénée, grade 90 | 0.05 % | 0.5 mg |
| huile de coton | Jusqu'à 100 % | jusqu'à 1 ml |

Les compositions huileuses à administration parentérale sont préparées selon un procédé comprenant les étapes suivantes :
i) chauffage du véhicule huileux biocompatible,
ii) optionnellement, addition de l'agent suspenseur ou de l'agent mouillant,
iii) refroidissement sous agitation,
iv) optionnellement, addition du second agent suspenseur ou de l'agent mouillant,
v) addition de l'anti-inflammatoire non stéroïdien portant une fonction acide,
vi) optionnellement addition de l'agent anti-infectieux,
vii) mélange sous agitation jusqu'à l'obtention d'une suspension homogène,
viii) répartition dans les emballages primaires,
ix) stérilisation par irradiation gamma à 25 kGy minimum.

L'invention sera mieux comprise à la lecture du complément de description qui suit et se réfère à des exemples de réalisation de compositions huileuses à administration parentérale conformes à l'invention, ainsi que des démonstrations de leurs propriétés particulières. Il doit être bien entendu, toutefois, que ces exemples ne sont donnés qu'à titre d'illustrations de l'invention et n'en constituent en aucune manière une limitation.

### EXEMPLE 1 - Composition huileuse à administration parentérale à 10% en poids/volume de kétoprofène

| Formule | | Quantité en p/V |
|---|---|---|
| Kétoprofène | | 10,0 % |
| Huile de coton | qsp | 100,0 % |

Le kétoprofène est dispersé sous agitation dans l'huile de coton jusqu'à l'obtention d'une suspension homogène.

La suspension est ensuite répartie en flacons de verre puis les flacons sont stérilisés par irradiation gamma à 25 kGy environ.

### EXEMPLE 2 - Composition huileuse à administration parentérale à 15% en poids/volume de kétoprofène

La composition de formule suivante est préparée :

| **Nom** | **% p/V** | **quantité pour 1 ml** |
|---|---|---|
| **Agent actif** | | |
| Kétoprofène | 15 % | 150 mg |

| **Excipients** | | |
|---|---|---|
| Oléate de sorbitan | 0.15 % | 1.5 mg |
| Phosphatidylcholine hydrogénée, grade 90 | 0.05 % | 0.5 mg |
| huile de coton | jusqu'à 100 % | jusqu'à 1 ml |

La totalité de l'huile de coton est chauffée à 100°C sous agitation lente. Lorsque la température est atteinte, le Phospholipon 90 H est dispersé sous agitation ; l'agitation est maintenue jusqu'à fusion totale du Phospholidon 90 H.

La préparation est refroidie jusqu'à 25°C puis le Montane 80 VG PHA est introduit.

Le kétoprofène est ensuite introduit dans le mélange sous agitation en maintenant l'agitation pendant 40 minutes environ en surveillant que la température reste à 20°C environ.

La suspension est répartie en flacons de verre. Les flacons sont stérilisés par irradiation gamma à 25 kGy environ.

### EXEMPLE 3 - Etude des paramètres chimiques et physiques de la suspension huileuse de l'exemple 2

Les paramètres suivants on été évalués et étudiés pour la suspension huileuse de kétoprofène obtenue selon le procédé décrit dans l'exemple 2.

### a) Etude de la granulométrie

Les mesures de la taille des particules de kétoprofène ont été réalisées à l'aide d'un granulomètre Laser Mastersizer 2000. La distribution de la taille des particules est :

| | D (v;0.1) | D (v;0.5) | D (v;0.9) |
|---|---|---|---|
| Kétoprofène (Lot n° 10126) | ≤ 2,4µm | ≤ 12,7µm | ≤ 44,8 µm |

| | | | |
|---|---|---|---|
| D (v,0.1) est la valeur du diamètre pour laquelle 10% de la distribution est intérieure ou égale ; D (v,0.5) est la valeur du diamètre médian pour laquelle 50% de la distribution est inférieure ou égale, et D (v,0.9) est la valeur du diamètre pour laquelle 90% de la distribution est inférieure ou égale. | | | |

### b) Etude de la stérilité

### - à temps zéro après l'irradiation

L'étude est conduite par filtration sur membrane conformément à la réglementation pharmaceutique en vigueur après validation.
Le produit de l'exemple 2 s'est révélé stérile.

### - après plusieurs utilisations

Une unité de ce produit peut être utilisée pour traiter un animal ou plusieurs animaux après un seul ou plusieurs soutirages de la composition huileuse dans un même temps ou à des temps différents pouvant s'étaler sur plusieurs mois. Une simulation de cette pratique vétérinaire a été conduite sur plusieurs flacons de 250 ml. Pour cela à temps zéro (t₀), à temps 1 mois (t_{1 mois}), à temps 2 mois (t_{2 mois}), et à temps 3 mois (t_{3 mois}), 50 ml ont été prélevés par flacon à l'aide d'une seringue équipée d'une aiguille, l'ensemble étant stérile, comme le ferait le vétérinaire. Pendant toute l'étude les flacons ont été conservés de manière habituelle pour ce genre de produit. L'étude de stérilité telle que présentée ci-dessus après irradiation a été conduite pour chacun des prélèvements effectués aux différents temps. Aucune contamination n'a été relevée ce qui montre que la composition est restée stérile au moins 3 mois.

### c) Etude de la remise en suspension et de la seringabilité

### - remise en suspension

Un test de centrifugation-remise en suspension a été conduit suivant la technique suivante : 16 g de suspension huileuse de suspension sont introduits dans un tube de 25 ml. Le tube est placé dans une centrifugeuse à 5000 tours/min à 25°C pendant 15 minutes. Le volume de sédiments et le temps de remise en suspension du culot de centrifugation sont déterminés. Après la détermination du pourcentage de sédimentation (rapport entre la hauteur du culot et la hauteur totale de la suspension dans le tube), le temps de remise en suspension est recherché par des agitations manuelles successives de 5 secondes du tube. La remise en suspension est considérée obtenue quand il n'y a plus de produit collé au fond du tube.

La suspension huileuse à 15% en poids/volume de kétoprofène selon l'invention décrite dans l'exemple 2, une suspension, de même formule que la suspension selon l'invention mais contenant en plus 1% en poids/volume d'alcool benzylique comme solvant secondaire facilitant la remise en suspension, et une suspension huileuse à 15% en poids/volume d'amoxicilline du commerce (Suramox®), prise comme référence car reconnue par les professionnels de la santé animale comme se remettant parfaitement en suspension, ont été comparées :

| | Suspension huileuse de Kétoprofène selon l'exemple 2 (Lot 10126) | Suspension huileuse de Kétoprofène contenant 1% d'alcool benzylique (Lot 11165) | Suspension huileuse d'Amoxicilline (Lot 32Y3) |
|---|---|---|---|
| Sédimentation | 79,2% | 78,3% | 74,7 % |
| Temps de remise en suspension | 50 sec | 70 sec | 40 sec |

Si la suspension huileuse de kétoprofène selon l'invention est équivalente à une suspension référence du marché, il n'en est plus de même lorsqu'un solvant est ajouté pour faciliter ladite remise en suspension.

### - Seringabilité

Le test de seringabilité, test qui permet d'évaluer l'aptitude à remplir et vider une seringue au travers d'une aiguille c'est-à-dire les forces à appliquer sur le piston d'une seringue équipée d'une aiguille de 14G pour aspirer 10 ml de suspension et expulser ces 10 mêmes millilitres de la seringue au travers de l'aiguille 14G, a été conduit sur la suspension huileuse à 15% (p/V) de kétoprofène selon l'invention décrite dans l'exemple 2, une suspension, de même formule que la suspension selon l'invention mais contenant en plus 1% (p/V) d'alcool benzylique comme solvant secondaire facilitant la remise en suspension, une suspension huileuse à 15% (p/V) d'amoxicilline du commerce (Suramox®), prise comme référence car reconnue par les professionnels de la santé animale comme se prélevant et s'injectant de manière satisfaisante, et une solution aqueuse commerciale à 10% (p/V) de kétoprofène et 1% d'alcool benzylique (Kétofen®) :

| | Suspension huileuse de Kétoprofène selon l'invention (Lot 10126) | Suspension huileuse de Kétoprofène contenant 1% d'alcool benzylique (Lot 11165) | Suspension huileuse d'Amoxicilline (Lot 32Y3) | Solution aqueuse de Ketoprofène contenant 1% d'alcool benzylique (Lot WO91101F) |
|---|---|---|---|---|
| Force pour aspirer 10 ml | 6,5 N | 5,5 N | 8,4 N | 11,8 N |
| Force pour expulser 10 ml | 8,4 N | 7,9 N | 34,1 N | 13,7 N |

En ce qui concerne la seringabilite, la suspension huileuse de kétoprofène selon l'invention est équivalente à une même suspension contenant 1% de solvant fluidisant ; elle se révèle supérieure à une référence du marché et même à une solution aqueuse du même actif connue pour pouvoir être injectée facilement.

### d) Etude de la pharmacocinétique du kétoprofène

Une étude de la pharmacocinétique de la composition huileuse conforme à l'invention du kétoprofène, a été réalisée chez les bovins par administration parentérale, voie intramusculaire.

Chacun des six animaux du groupe, d'un poids compris entre 239 et 312 kg, le groupe étant composé de bovin de race Croisé, Limousine, Blonde d'Aquitaine et des deux sexes, a reçu 3 mg de kétoprofène par kg de poids vif de la composition huileuse décrite dans l'exemple 2 par voie intramusculaire.

10 ml de sang ont été prélevés à chaque temps de prélèvement dans des tubes d'héparine de lithium. Les prélèvements ont été réalisés aux temps suivants : avant le traitement et à 5 min, 10 min, 20 min, 30 min, 40 min, 60 min et à 1.5h, 2h, 3h, 4h, 6h, 8h, 12h et 24h après le traitement par la composition huileuse conforme à l'invention.

L'analyse des échantillons a été conduite selon les techniques chromatographiques, CLHP avec détection UV, bien connues de l'homme de métier.

Les paramètres pharmacocinétiques obtenus soit le Cmax (concentration plasmatique maximale), le Tmax (temps pour atteindre la concentration plasmatique maximale), et le temps de demi-vie apparent t_{half} sont représentés ci- dessous.

| | C ₘₐₓ (ng.ml⁻¹) | Tmax (h) | Apparent t_{half} (h) |
|---|---|---|---|
| Exemple 2 | 6164 ± 2031 | 0.72 ± 0.23 | 2.42 |
| Comforion® données de la littérature formulation aqueuse contenant : | 9700 | 0.5 | 2.5 |
| - Kétoprofène (100 mg/ml) | | | |
| - Arginine | | | |
| - acide citrique monohydraté | | | |
| - alcool benzylique. | | | |

Ces résultats montrent que la composition huileuse de l'exemple 2, conforme à l'invention, comparativement aux données de la littérature (réf : Summary of Products Characteristics - http://www.hevra.org/vmri_spc/spc.asp?Product_Identifier=FI/V/0101/001) est similaire à une solution aqueuse sur le plan pharmacocinétique.

### e) Association extemporanée avec EXCENEL® RTU

La composition huileuse de l'exemple 1 est mélangée avec de EXCENEL® RTU à volume égal. EXCENEL® RTU est une suspension huileuse d'antibiotique commercialisée dans le domaine vétérinaire. Il est observé une parfaite compatibilité entre les deux véhicules huileux. Aucune démixtion n'est observée, ni d'émulsion comme il se passerait si il y avait une présence d'eau. Après agitation la suspension composée est parfaitement homogène ne présentant aucun agglomérat ou masse visqueuse. La suspension passe parfaitement au travers d'un orifice comme une aiguille pour injection.

### EXEMPLE 4 - Composition huileuse à administration parentérale à 15% en p/V de kétoprofène et 5% en p/V de ceftiofur

La composition de formule suivante est préparée :

| **Nom** | **% p/V** | **quantité pour 1 ml** |
|---|---|---|
| **Agents actifs** | | |
| Ceftiofur (sous forme de Chlorhydrate) | 5% | 50 mg |
| Kétoprofène | 15 % | 150 mg |

| **Excipients** | | |
|---|---|---|
| Oleate de sorbitan | 0.15 % | 1.5 mg |
| Pphosphatidylcholine hydrogénée, grade 90 | 0.05 % | 0.5 mg |
| huile de coton | Jusqu'à 100 % | jusqu'à 1 ml |

La totalité de l'huile de coton est chauffée à 100°C sous agitation lente. Lorsque la température est atteinte, le Phospholipon 90 H est dispersé sous agitation. Maintenir l'agitation jusqu'à fusion totale du Phospholidon 90 H. Laisser refroidir la préparation jusqu'à 25°C et introduire le Montane 80 VG PHA. Introduction du chlorhydrate de Ceftiofur sous agitation en maintenant l'agitation pendant 20 minutes environ en surveillant que la température reste à 20°C environ. Introduction du Kétoprofène sous agitation en maintenant l'agitation pendant 40 minutes environ en surveillant que la température reste à 20°C environ. La suspension est répartie en flacons de verre. Les flacons sont stérilisés par irradiation gamma à 25 kGy environ.

### EXEMPLE 5 - Etude des paramètres chimiques et physiques de la suspension huileuse de l'exemple 4

Les paramètres suivants on été évalués et étudiés pour la suspension huileuse de kétoprofène et Ceftiofur (lot 10092), obtenue selon le procédé décrit dans l'exemple 4.

### a) Etude de la granulométrie

Les mesures de la taille des particules de kétoprofène/Ceftiofur ont été réalisées à l'aide d'un granulomètre Laser Mastersizer 2000. La distribution de la taille des particules est :

| | D (v;0.1) | D (v;0.5) | D (v;0.9) |
|---|---|---|---|
| Lot n° MER 10092 | ≤ 1.5 µm | ≤ 5.4 µm | ≤ 22,2 µm |

### b) Etude de la stérilité

### - à temps zéro après l'irradiation

L'étude est conduite par filtration sur membrane conformément à la réglementation pharmaceutique en vigueur après validation.

Le produit de l'exemple 4 s'est révélé stérile.

### - après plusieurs utilisations

Une unité de ce produit peut être utilisée pour traiter un animal ou plusieurs animaux après un seul ou plusieurs soutirages de la composition huileuse dans un même temps ou à des temps différents pouvant s'étaler sur plusieurs mois. Une simulation de cette pratique vétérinaire a été conduite sur plusieurs flacons de 250 ml. Pour cela à temps zéro (t₀), à temps 1 mois (t_{1 mois}), à temps 2 mois (t_{2 mois}), et à temps 3 mois (t_{3 mois}), 50 ml ont été prélevés par flacon à l'aide d'une seringue équipée d'une aiguille, l'ensemble étant stérile, comme le ferait le vétérinaire. Pendant toute l'étude les flacons ont été conservés de manière habituelle pour ce genre de produit. L'étude de stérilité telle que présentée ci-dessus après irradiation a été conduite pour chacun des prélèvements effectués aux différents temps. Aucune contamination n'a été relevée ce qui montre que la composition est restée stérile au moins 3 mois.

### c) Etude de la seringabilité

Le test de seringabilité, test qui permet d'évaluer l'aptitude à remplir et vider une seringue au travers d'une aiguille c'est-à-dire les forces à appliquer sur le piston d'une seringue équipée d'une aiguille de 14G pour aspirer 10 ml de suspension et expulser ces 10 mêmes millilitres de la seringue au travers de l'aiguille 14G, a été conduit sur la suspension huileuse à 15% en poids/volume de kétoprofène et 5% en poids/volume de Ceftiofur selon l'invention décrite dans l'exemple 4, et une suspension huileuse à 15% en poids/volume d'amoxicilline du commerce (Suramox®), prise comme référence car reconnue par les professionnels de la santé animale comme se prélevant et s'injectant de manière satisfaisante, et une solution aqueuse commerciale à 10% (p/V) de kétoprofène et 1% d'alcool benzylique (Kétofen®) :

| | Suspension huileuse de Kétoprofène/Ceftiofur selon l'exemple 4 | Suspension huileuse d'Amoxicilline | Solution aqueuse de Kétoprofène contenant 1% d'alcool benzylique |
|---|---|---|---|
| | (Lot 10092) | (Lot 32Y3) | (Lot WO91101 |
| Force pour aspirer 10 ml | 5,8 N | 8,4 N | 11,8 N |
| Force pour expulser 10 ml | 8,1 N | 34,1 N | 13,7N |

La suspension huileuse de kétoprofène/Ceftiofur selon l'invention est bien meilleure que la référence du marché et même qu'une solution aqueuse de kétoprofène connue pour s'injecter facilement.

### d) Etude de la pharmacocinétique du kétoprofène en présence ou non de ceftiofur (comparaison des exemples 2 et 4)

L'étude de la pharmacocinétique a été réalisée chez les bovins par voie intramusculaire pour les compositions huileuses conformes à l'invention décrite dans les exemples 2 et 4. Chacun des animaux de chacun des 2 groupes de 6 bovins, d'un poids compris entre 239 et 312 kg, les 2 groupes étant composé de bovin de race Croisé, Limousine, Blonde d'Aquitaine et des deux sexes, a reçu :
- 3 mg de kétoprofène par kg de poids vif de la composition huileuse décrite dans l'exemple 2 pour le premier groupe,
- 3 mg de kétoprofène et 1 mg de ceftiofur par kg de poids vif de la composition huileuse décrite dans l'exemple 4 pour le second groupe.

10 ml de sang ont été prélevés à chaque temps de prélèvement dans des tubes d'héparine de lithium. Les prélèvements ont été réalisés aux temps suivants : avant le traitement et à 5 min, 10 min, 20 min, 30 min, 40 min, 60 min et à 1.5h, 2h, 3h, 4h, 6h, 8h, 12h et 24h après le traitement par la composition huileuse conforme à l'invention.

L'analyse des échantillons a été conduite selon les techniques chromatographiques, CLHP avec détection UV, bien connues de l'homme de métier.

Les paramètres pharmacocinétiques obtenus soit le Cmax (concentration plasmatique maximale), l'AUClast (Aire sous la courbe jusqu'à la dernière concentration mesurée, et le temps de demi-vie apparent t_{half} sont représentés ci dessous.

**Pour le kétoprofène :**

| Groupe traité avec | Cₘₐₓ (ng.mL⁻¹) | AUClast (ng.h.mL⁻¹) | Apparent t_{half}^{a} (h) |
|---|---|---|---|
| Exemple 2 | 6164 ± 2031 | 19571 ± 3705 | 2.42 |
| Exemple 4 | 5546 ± 1583 | 27186 ± 4680 | 3.75 |

| | | | |
|---|---|---|---|
| (a) moyenne harmonique | | | |

Les données du tableau montrent que l'adjonction d'un autre actif a légèrement modifié la biodisponibilité du kétoprofène (comme reflété par l'AUClast).

### EXEMPLE 6 - Etude clinique de la suspension huileuse de l'exemple 4

Cette étude comparative des effets antipyrique et antiinflammatoire du kétoprofène a été réalisée chez les bovins.

Chacun des animaux de chacun des 3 groupes de 8 bovins, d'un poids compris entre 239 et 312 kg, les 3 groupes étant composé de bovin de race Croisé, Limousine, Blonde d'Aquitaine et des deux sexes, a reçu par injection intramusculaire :
- 3 mg de kétoprofène par kg de poids vif de la composition huileuse décrite dans l'exemple 4 pour le premier groupe,
- 3 mg de kétoprofène par kg de poids vif de Ketofen®, solution aqueuse, pour le deuxième groupe, et
- une solution saline comme témoin négatif.

1 heure avant le traitement par l'une des 3 compositions, chacun des bovins a reçu par voie intraveineuse une solution d'endotoxines d'*Escherichia coli* (055 :B5) à raison de 40.6 ± 0.9 ng.kg⁻¹.

Les paramètres, température rectale et le taux de thromboxane B2 ont été suivis pendant 10 heures et montrent que les effets antipyrique et anti-inflammatoire de la suspension huileuse conforme à l'invention ne sont pas différents de ceux de la solution aqueuse de kétoprofène.

### EXEMPLE 7 - Composition huileuse à administration parentérale à 10% en poids/volume de kétoprofène et 5% en poids/volume d'amoxicilline

| Formule | | Quantité en p/V |
|---|---|---|
| Kétoprofène | | 10,0% |
| Amoxicilline anhydre | | 5,0 % |
| Acide stéarique | | 1.5 % |
| Stéarate d'Aluminium | | 1.3 % |
| Huile de coton | qsp | 100,0 % |

### EXEMPLE 8 - Composition huileuse à administration parentérale à 10% en poids/volume d'ibuprofène et 4% en poids/volume de cephalexine

### Exemple de référence

| Formule | | Quantité en p/V |
|---|---|---|
| Ibuprofène | | 10,0 % |
| Céphalexine (sous forme monohydrate) | | 4,0 % |
| Mygliol 840 | qsp | 100,0 % |

## Revendications

1. Composition huileuse d'anti-inflammatoire non stéroïdien (AINS) pour une administration parentérale comprenant :
- au moins un AINS portant une fonction acide ; et
- un véhicule huileux biocompatible ;
ladite composition étant **caractérisée en ce que** :
- ledit AINS portant une fonction acide est le ketoprofène ;
- ledit AINS est sous forme d'acide libre ;
- ledit AINS répond aux spécifications suivantes : un diamètre médian D (v,0.5), valeur pour laquelle 50% de la distribution est inférieure ou égale, de 25 µm et une valeur D (v,0.9), pour laquelle 90% de la distribution est inférieure ou égale, de 80 µm ; les valeurs des D (v,0.5) et D (v,0.9) étant mesurées par une granulomètre Laser Mastersizer 2000 ; et
- ladite composition ne contient aucun agent liquide ajouté capable de solubiliser ledit AINS.

2. Composition selon la revendication 1, **caractérisée en ce qu'**elle comprend entre 1 et 30% en poids/volume d'au moins de kétoprofène.

3. Composition selon l'une quelconque des revendications précédentes, **caractérisée en ce que** ledit agent liquide ajouté capable de solubiliser ledit composé anti-inflammatoire non stéroïdien est choisi parmi l'eau, le propylène glycol, le glycérol, le polyéthylène glycol, des alcools en C₁ à C₆, l'alcool benzylique et les autres alcools aromatiques.

4. Composition selon l'une quelconque des revendications précédentes, **caractérisée en ce qu'**elle comprend en outre au moins un agent mouillant étant choisi parmi : le monostéarate de glycérol, les huiles de ricin polyoxyéthylénées, les esters d'acides gras polyoxyéthylénés et de sorbitan, les esters d'acides gras et de sorbitan, la lécithine et ses dérivés de soja ou d'œuf comme par exemple la phosphatidylcholine, la phosphatidylcholine hydrogénée, la lysophosphatidylcholine, la lysophosphatidylcholine hydrogénée, ainsi que leur combinaison.

5. Composition selon l'une quelconque des revendications précédentes, **caractérisée en ce qu'**elle a la composition suivante :
| **Nom** | **% m/V** | **quantité pour 1 ml** |
|---|---|---|
| **Agent actif** | | |
| Kétoprofène | 15 % | 150 mg |
| **Excipients** | | |
|---|---|---|
| Oleate de sorbitan | 0.15 % | 1.5 mg |
| Phosphatidylcholine hydrogénée, grade 90 | 0.05 % | 0.5 mg |
| Huile de coton | jusqu'à 100 % | jusqu'à 1 ml |

6. Composition selon l'une quelconque des revendications précédentes pour utilisation dans le traitement de l'inflammation et/ou de la douleur et/ou de la fièvre des animaux d'élevage et des animaux de compagnie.

7. Composition selon l'une quelconque des revendications 1 à 5, **caractérisée en ce qu'**elle comprend en outre au moins un agent anti-infectieux en suspension dans ledit véhicule huileux.

8. Composition selon la revendication 7, **caractérisée en ce que** ledit agent anti-infectieux est un antibiotique bêta-lactamine choisi parmi les benzylpénicillines, les aminopénicillines ou les céphalosporines.

9. Composition selon la revendication 7 ou la revendication 8, **caractérisée en ce que** ladite composition comprend entre 1 et 20 % en poids/volume d'agent anti-infectieux.

10. Composition selon l'une quelconque des revendications 7 à 9, **caractérisée en ce qu'**elle a la composition suivante :
| **Nom** | **% m/V** | **quantité pour 1 ml** |
|---|---|---|
| **Agents actifs** | | |
| Ceftiofur (sous forme de chlorhydrate) | 5% | 50 mg |
| Kétoprofène | 15 % | 150 mg |
| **Excipients** | | |
|---|---|---|
| Oleat de sorbitan | 0.15 % | 1.5 mg |
| Phosphatidylcholine hydrogénée, grade 90 | 0.05 % | 0.5 mg |
| Huile de coton | Jusqu'à 100 % | jusqu'à 1 ml |

11. Composition selon l'une quelconque des revendications 7 à 10, pour utilisation dans le traitement des maladies infectieuses des animaux d'élevage tels que les bovins, ovin et caprins ou des animaux de compagnies tels que les chiens et chats.

12. Procédé de préparation d'une composition selon l'une quelconque des revendications 1 à 5 ou 7 à 11, comprenant les étapes suivantes :
i) chauffage du véhicule huileux biocompatible,
ii) optionnellement, addition de l'agent suspenseur ou de l'agent mouillant,
iii) refroidissement sous agitation,
iv) optionnellement, addition du second agent suspenseur ou de l'agent mouillant,
v) addition du kétoprofène,
vi) optionnellement, addition de l'agent anti-infectieux,
vii) mélange sous agitation jusqu'à l'obtention d'une suspension homogène,
viii) répartition dans les emballages primaires,
ix) stérilisation par irradiation gamma à 25 kGy minimum.

## Patentansprüche

1. Ölige Zusammensetzung eines nichtsteroidalen Entzündungshemmers (NSAR) für eine parenterale Verabreichung, umfassend:
- mindestens einen NSAR mit einer Säurefunktion; und
- ein biokompatibles öliges Vehikel;
wobei die Zusammensetzung **dadurch gekennzeichnet ist, dass**:
- der NSAR mit einer Säurefunktion Ketoprofen ist;
- der NSAR in Form von freier Säure vorliegt;
- der NSAR den folgenden Spezifikationen genügt: einen Mediandurchmesser D(v,0,5) von 25 µm als Wert, bei dem 50% der Verteilung kleiner oder gleich ist, und einen D(v,0,9)-Wert von 80 µm, bei dem 90% der Verteilung kleiner oder gleich ist; wobei der D(v,0,5)- und der D(v,0,9)-Wert durch ein Laser-Granulometer Mastersizer 2000 gemessen werden; und
- die Zusammensetzung keinen hinzugegebenen flüssigen Wirkstoff enthält, der imstande ist, den NSAR aufzulösen.

2. Zusammensetzung nach Anspruch 1, **dadurch gekennzeichnet, dass** sie zwischen 1 und 30 Gewichts-/Volumen-% an mindestens Ketoprofen umfasst.

3. Zusammensetzung nach einem der vorstehenden Ansprüche, **dadurch gekennzeichnet, dass** der hinzugegebene flüssige Wirkstoff, der imstande ist, die nichtsteroidale entzündungshemmende Verbindung aufzulösen, aus Wasser, Propylenglycol, Glycerin, Polyethylenglycol, C₁- bis C₆-Alkoholen, Benzylalkohol und den anderen aromatischen Alkoholen ausgewählt ist.

4. Zusammensetzung nach einem der vorstehenden Ansprüche, **dadurch gekennzeichnet, dass** sie weiter mindestens ein Benetzungsmittel umfasst, das ausgewählt ist aus: Glycerinmonostearat, polyoxyethylenierten Rizinusölen, polyoxyethylenierten Sorbitanfettsäureestern, Sorbitanfettsäureestern, Lecithin und seinen Soja- oder Eiderivaten, wie zum Beispiel Phosphatidylcholin, hydriertes Phosphatidylcholin, Lysophosphatidylcholin, hydriertes Lysophosphatidylcholin, sowie deren Kombination.

5. Zusammensetzung nach einem der vorstehenden Ansprüche, **dadurch gekennzeichnet, dass** sie die folgende Zusammensetzung besitzt:
| **Bezeichnung** | **% m/V** | **Menge für 1 ml** |
|---|---|---|
| **Aktiver Wirkstoff** | | |
| Ketoprofen | 15% | 150 mg |
| **Hilfsstoffe** | | |
|---|---|---|
| Sorbitanoleat | 0,15% | 1,5 mg |
| hydriertes Phosphatidylcholin, Stufe 90 | 0,05 % | 0,5 mg |
| Baumwollöl | bis auf 100 % | bis auf 1 ml |

6. Zusammensetzung nach einem der vorstehenden Ansprüche zur Verwendung bei der Entzündungs- und/oder der Schmerz- und/oder der Fieberbehandlung von Nutztieren und von Haustieren.

7. Zusammensetzung nach einem der Ansprüche 1 bis 5, **dadurch gekennzeichnet, dass** sie weiter mindestens einen Wirkstoff gegen Infektionskrankheiten in Suspension im öligen Vehikel umfasst.

8. Zusammensetzung nach Anspruch 7, **dadurch gekennzeichnet, dass** der Wirkstoff gegen Infektionskrankheiten ein Beta-Lactam-Antibiotikum ist, das ausgewählt ist aus Benzylpenicillinen, Aminopenicillinen oder Cephalosporinen.

9. Zusammensetzung nach Anspruch 7 oder Anspruch 8, **dadurch gekennzeichnet, dass** die Zusammensetzung zwischen 1 und 20 Gewichts-/Volumen-% an Wirkstoff gegen Infektionskrankheiten umfasst.

10. Zusammensetzung nach einem der Ansprüche 7 bis 9, **dadurch gekennzeichnet, dass** sie die folgende Zusammensetzung besitzt:
| **Bezeichnung** | **% m/V** | **Menge für 1 ml** |
|---|---|---|
| **Aktive Wirkstoffe** | | |
| Ceftiofur (in Form von Chlorhydrat) | 5% | 50 mg |
| Ketoprofen | 15% | 150 mg |
| **Hilfsstoffe** | | |
|---|---|---|
| Sorbitanoleat | 0,15% | 1,5 mg |
| hydriertes Phosphatidylcholin, Stufe 90 | 0,05 % | 0,5 mg |
| Baumwollöl | bis auf 100 % | bis auf 1 ml |

11. Zusammensetzung nach einem der Ansprüche 7 bis 10, zur Verwendung bei der Behandlung von Infektionskrankheiten bei Nutztieren wie Rindern, Schafen und Ziegen oder bei Haustieren wie Hunden und Katzen.

12. Verfahren zur Herstellung einer Zusammensetzung nach einem der Ansprüche 1 bis 5 oder 7 bis 11, umfassend die folgenden Schritte:
i) Erwärmen des biokompatiblen öligen Vehikels,
ii) gegebenenfalls Zugabe des Suspensionsmittels oder des Benetzungsmittels,
iii) Abkühlen unter Rühren,
iv) gegebenenfalls Zugabe des zweiten Suspensionsmittels oder des Benetzungsmittels,
v) Zugabe von Ketoprofen,
vi) gegebenenfalls Zugabe des Wirkstoffs gegen Infektionskrankheiten,
vii) Mischen unter Rühren bis zum Erhalt einer homogenen Suspension,
viii) Aufteilen in die Primärverpackungen,
ix) Sterilisation durch Gammabestrahlung bei mindestens 25 kGy.

## Claims

1. Oily composition of nonsteroidal anti-inflammatory drug (NSAID) for parenteral administration comprising:
- at least one NSAID bearing an acidic function; and
- a biocompatible oily vehicle;
said composition being **characterised in that**:
- said NSAID bearing an acidic function is ketoprofen;
- said NSAID is in free acid form,
- said NSAID meets the following specifications: a median diameter D (v,0.5), value of which 50% of the distribution is inferior or equal, of 25µm and a value D (v,0.9), for which 90% of the distribution is inferior or equal, of 80µm; the values of D (v,0.5) and D (v,0.9) being measured by a Laser Mastersizer 2000 granulometer; and
- said composition does not contain an added liquid agent capable of solubilising said NSAID.

2. Composition according to claim 1, **characterised in that** it comprises between 1 and 30% by weight/volume of at least ketoprofen.

3. Composition according to any one of the preceding claims, **characterised in that** said added liquid agent capable of solubilising said nonsteroidal anti-inflammatory compound is chosen from among water, propylene glycol, glycerol, polyethylene glycol, C₁ to C₆ alcohols, benzyl alcohol, and other aromatic alcohols.

4. Composition according to any one of the preceding claims, **characterised in that** it further comprises at least one wetting agent chosen from among: glycerol monostearate, polyoxyethylated castor oils, polyoxyethylated fatty acid and sorbitan esters, fatty acid and sorbitan esters, lecithin and its soy or egg derivatives for example phosphatidylcholine, hydrogenated phosphatidylcholine, lysophosphatidylcholine, hydrogenated lysophosphatidylcholine, as well as their combination.

5. Composition according to any one of the preceding claims, **characterised in that** it has the following composition:
| **Name** | **% m/V** | **Quantity per 1ml** |
|---|---|---|
| **Active agent** | | |
| Ketoprofen | 15% | 150mg |
| **Excipients** | | |
|---|---|---|
| Sorbitan Oleate | 0.15% | 1.5mg |
| Grade 90 hydrogenated phosphatidylcholine | 0.05% | 0.5mg |
| Cottonseed oil | up to 100% | up to 1ml |

6. Composition according to any one of the preceding claims for use in the treatment of inflammation and/or pain and/or fever in livestock and pets.

7. Composition according to any one of claims 1 to 5, **characterised in that** it further comprises an anti-infective agent in suspension in said oily vehicle.

8. Composition according to claim 7, **characterised in that** said anti-infective agent is a beta-lactam antibiotic chosen from among benzylpenicillins, aminopenicillins, or cephalosporins.

9. Composition according to claim 7 or claim 8, **characterised in that** said composition comprises between 1 and 20% of the anti-infective agent by weight/volume.

10. Composition according to any one of claims 7 to 9, **characterised in that** it has the following composition:
| **Name** | **% m/V** | **Quantity per 1ml** |
|---|---|---|
| **Active agents** | | |
| Ceftiofur (in chlorhydrate form) | 5% | 50mg |
| Ketoprofen | 15% | 150mg |
| **Excipients** | | |
|---|---|---|
| Sorbitan Oleate | 0.15% | 1.5mg |
| Grade 90 hydrogenated phosphatidylcholine | 0.05 % | 0.5mg |
| Cottonseed oil | Up to 100% | up to 1ml |

11. Composition according to any one of claims 7 to 10, for use in the treatment of infectious diseases in livestock such as cattle, sheep and goats, or pets such as cats and dogs.

12. Method for preparing a composition according to any one of claims 1 to 5 or 7 to 11, comprising the following steps:
i) heating of the biocompatible oily vehicle,
ii) optionally, adding the suspending agent or the wetting agent,
iii) stirred cooling,
iv) optionally, adding the second suspending or wetting agent,
v) adding the ketoprofen,
vi) optionally, adding the anti-infective agent,
vii) stir-mixing until obtaining a homogeneous suspension,
viii) distributing in primary packaging,
ix) sterilising via Gamma irradiation at a minimum of 25kGy.
